# EUROPEAN PATENT SPECIFICATION

(11) **EP 0 519 240 B1**
(45) Date of publication and mention of the grant of the patent: **19.07.1995**
(21) Application number: 92108911.6
(22) Date of filing: 27.05.1992
(51) Int. Cl.: A61M 39/00, A61J 1/00

(54) **Syringe with a closed sealable tube**
Medizinische Spritze mit geschlossenem abdichtbarem Schlauch
Seringue avec tuyeau fermé scellable

(30) Priority: 21.06.1991 US 719728
(43) Date of publication of application: 23.12.1992
(73) Proprietor: NPBI Nederlands Produktielaboratorium voor Bloedtransfusieapparatuur en Infusievloeistoffen B.V., NL-7881 HM Emmer Compascuum (NL)
(72) Inventor: Van der Heiden, Johannes, NL-9718 DK Groningen (NL); Hilbrink, Hubertus Eduard, NL-7827 BL Emmen (NL)
(74) Representative: Masch, Karl Gerhard, Dr.

(56) References cited:
- FR-A- 2 400 911
- US-A- 3 277 894
- US-A- 4 369 779

## Description

Syringes are widely used in the medical field for the administration of sterile fluids.
The fluids administered to patients by means of a syringe may be plain physiological solutions such as physiological saline and dextrose 5% - to compensate for fluid losses-, or they may contain a medicament - to treat a disease-, or they may be a bloodproduct - to compensate for the shortage or loss of a bloodcomponent- or they may contain nutrients.
These fluids may be administered with a syringe by direct iv push injection or as an infusion. Generally, if the fluid must be administered slowly in more than 5 to 10 minutes, it is referred to as an infusion. For that purpose, special pumps are available that can accommodate a syringe.

Syringes are commercially available in sizes ranging from smaller than 1 ml to over 50 ml from many different manufacturers. The syringes with the sizes of approximately 1 ml may be intended for specific purposes such as the administration of e.g. insulin or tuberculin and may constitute an integrated needle. Syringes constitute a plunger with a piston that may be made of natural or silicone rubber, placed in a barrel and lubricated with a lubricant such as polydimethyl siloxane. The barrel is usually manufactured from plastics like e.g. polypropylene, polystyrene or styrene/acrylonitrile copolymer. The barrel is fitted with graduation lines and with a nozzle. The nozzle usually constitutes a male liner or luer-lock connector and may be fitted with a nozzle cap to guard against accidental touch-contamination. After packaging, this syringe is sterilized (usually by ETO-gas or by gamma- or beta-radiation) and supplied ready for -single- use.

The requirements that syringes must meet are described in many standards, especially ISO. ISO sets standards for a.o. the materials of the syringe, the manufacture, accuracy, performance etc. Also, ISO -among other organizations- sets standards for the male luer or luer-lock connector that is fitted on the syringe. ISO also sets standards for female liner connectors, thereby guaranteeing a leaktight fit of the connector on the syringe with any device with a female luer connector.

The state of the art in administering a sterile fluid in a syringe to a patient is to first fill the syringe with the solution to be administered.
This solution may be contained in a container made of a rigid material such as glass, of a semi-rigid material such as e.g. polyethylene or polypropylene or of a flexible material such as polyvinylchloride. These containers usually constitute a port with a rubber membrane that can be punctured with a needle to introduce in or withdraw fluid from the container.

To maintain the sterility of both the solution to be sampled and the solution in the syringe, sampling is preferably performed under aseptic conditions in a LAF-cabinet by personnel trained in aseptic techniques and wearing special clothing.
First, a needle is placed on the syringe. Needles usually have a female luer connector and therefore can be connected to the male connector of the syringe.
Then, with the needle, the rubber membrane of the container is punctured. By applying force to the plunger, the required volume of solution is drawn into the syringe. Air is expelled from the syringe. Subsequently, the needle is withdrawn from the container and the fluid may be administered to the patient immediately if preparation takes place at the bedside, or -after removal of the needle- a cap may be put on the nozzle of the syringe.
After capping, the syringe may be labelled and packaged and sent to the ward for administration to the patient.
If the solution is intended for direct intravenous push injection, after removal and disposal of the cap, a needle is placed on the nozzle again, and the solution may be injected into a vein or may be injected into a running administration system.
If the solution is intended for slow administration over more than 5 min. it usually is administered with the aid of a syringe pump. For that purpose, the cap is removed from the syringe, disposed of and the nozzle is connected to a tube with a female liner connector. The tube is connected to the administration system of the patient with a needle via an injection port or a gum rubber injection site.
For the slow administration of sterile fluids in a volume up to 50 ml, the use of a syringe with a syringe pump is often preferred for reasons of accuracy, reliability, ease of use and the presence of alarms on the pumps.

Although the use of a syringe may be the preferred way for the administration of small volumes of up to 50 ml of medical fluids to patients, it has some serious draw-backs.
The main drawback results from the fact that the syringe must be filled in an open system, i.e. with the aid of a needle in an aseptic procedure.
In an open, aseptic procedure, foreign material may be introduced into the fluid in the syringe as well as in the fluid in the container. Also, in such a procedure, there is a high incidence of leakage and spillage due to needle drops and aerosols.
During filling, micro-organisms may accidentally be introduced into the syringe or into the container from which the syringe is being filled. For that reason, sterile fluids that have been sampled - in this case both the fluid in the syringe and the fluid in the container from which the syringe was filled- usually are regarded as being contaminated and have a limited expiry. Depending upon the nature of the product, the storage conditions and the policy of the hospital, the stability period for both the fluid in the syringe and the fluid in the container is limited to 1 to 7 days.
However, this limited expiry period itself does not preclude the microbiological contamination of the fluid in the syringe nor of the fluid in the container that is being sampled.
Another drawback of this method of filling a syringe is that a needle is required for the procedure. Use of a needle frequently leads to needle sticks. Also, the needle may accidentally cut through the wall of the IV container.
Use of a needle may lead to coring or laceration of the rubber membrane of the IV container and thus to particulate contamination of the IV fluid. Finally, the use of a needle requires considerable force to fill the syringe, as the needle presents the main constriction in the system.
The use of a needle -and thus an open system- may also lead to contamination of the environment with the product that is being filled in the syringe. This contamination may occur through needle drips, aerosols that are formed, spillage and leakage.

The problems that result from the use of a needle in an open, aseptic system to fill the syringe will be illustrated with 3 examples illustrating the consequences of contamination of the fluid in the syringe, contamination of the fluid in the container and contamination of the environment with the fluid.

Example 1: Contamination of the fluid in the syringe.
Generally, a considerable number of patients in hospitals acquire an infection. Syringes that were contaminated during sampling contribute to this phenomenon. As the roomtemperature in hospitals is generally high and many fluids may accommodate the growth of micro-organisms (e.g. parenteral nutrition and dextrose injections) and administration may take place over a number of hours, these solutions in syringes may become heavily contaminated during use.

Example 2: Contamination of the fluid in the container.
Some neonates need blood or bloodproducts immediately after birth. This must be administered slowly and in a small volume and therefore administration via a syringe and a pump is the preferred method of administration. For that purpose, blood is drawn into the syringe from a standard donation.
After sampling, for microbiological reasons the storage period of blood products is restricted to 24 hours. Thus, the original donation is discarded after 24 hours.
Therefore, if the child needs another transfusion on one or more of the next days, the blood must be taken from another donation. As the average child in this situation needs approximately 7 transfusions, this means that this child will be exposed to the blood of 7 different donors. The higher the donor-exposure however, the higher is the chance on the transmission of virus's like HIV, CMV and hepatitis, and on the occurrence of transfusion reactions which may consist of fever, chills, hypersensitivity reactions etc.

Also, the use of the blood of 7 different donors, means that compatability testing needs to be performed 7 times; with each new donation.
This example shows, that the limited expiry of a product that is sampled in an open system may itself be the cause of a new, medically undesirable situation for a patient.
If, however, the original blood donation could be sampled with a sterile technique in a closed system, this unit of blood could be used for all the transfusions the child would need. This would reduce donor-exposure to 1 and greatly reduce the chances on occurrence of transfusion reactions and the transmittance of virus's.
At the same time, use of such a technique could prevent microbial contamination of the transfusion product in the syringe as was discussed in example 1 and could thus reduce the -generally very high- infection rate with these patients.

Example 3 : Contamination of the environment.
Many cytotoxic drugs are preferably administered with the aid of a syringe and a syringe pump.
For that purpose, first the syringe must be filled with the drug solution. As most of these cytotoxic drugs are themselves carcinogenic and therefore hazardous, the reconstitution and dilution of these substances is usually performed under strict procedures based upon e.g. the guidelines issued by the American Society of Hospital Pharmacists or based upon local rules and regulations.
These usually comprise working in a special cabinet with filtered air, wearing special protective clothing and special rules for handling of the product and of the waste that is generated during reconstitution.
The drug may either be in a liquid form or in a powdered form and may be contained within a ampoule or within a glass vial with a rubber stopper.
If the drug is in a powdered form, first it must be reconstituted.
After reconstitution, the soluted drug is generally diluted in an IV container.
After dilution, the solution is drawn into the syringe from which it will be administered. A needle is put on the syringe and the required volume of the solution is drawn into the syringe. After withdrawal of the needle from the container, the needle is removed from the syringe. The liner connector on the syringe may be capped.
For administration, the cap is removed and the syringe is placed in a pump and is connected to the IV administration system of the patient.

The use of this method of working is associated with a considerable chance of leakage, drips etc. Especially the use of the needle during preparation and the removal of the protective cap carry the risk of inadvertent exposure to these hazardous, cytotoxic drugs.

The examples given above serve solely as an illustration of the problems that are encountered due to the use of an open system to fill a solution in a syringe but are in no way intended to limit the scope of the present invention to these examples.

Several techniques have been described that are capable of forming open fluid communication in a closed system. To these techniques is often refered under the name of sterile docking.
Such techniques may employ special connectors that can be sterilely welded such as described in U.S. patent no's 4.157.723 and 4.611.643 to Baxter Co.
Most of these sterile docking techniques, however, make use of a simple piece of sealable tubing that is closed on one end and is in communication with a component on the other end. This tubing may be of a material like e.g. PVC. The components that need to be coupled must both be fitted with such a piece of tubing. These tubes are put in an apparatus and are docked. Such systems are described a.o. in U.S. patents no's 4.369.799 and 4.619.642 and 4.737.214.
An apparatus for making sterile connections is marketed in the U.S. by the Haemonetics company.
A system to use sterile docking to connect a bag containing a sterile CAPD fluid to the intraperitoneal catheter of a patient is marketed in Europe by the Gambro company.
Sterile docking is used in some bloodbanks for the production of bloodcomponents. For this purpose, sterile fluids in flexible containers with a PVC tube for sterile docking are marketed in the Netherlands by NPBI.

It is an object of the present invention to provide an improved apparatus and method for the filling of medical fluids in a syringe in which many of the drawbacks of the methods as mentioned above, are avoided.

More specifically, an object of this invention is to provide an apparatus and a method for filling a medical solution that is contained in a container, in a syringe applying a closed system, without a need for the use of needles.

The invention consists of an apparatus and a method for filling a syringe with a medical solution in a closed system via sterile docking.

These objects and others which will be apparent hereinafter are attained, in accordance with the invention in a sterilized apparatus for medical use consisting of a syringe that is fitted with a sealable tube, said sealable tube being closed on one end and in communication with said syringe on the other end.

This communication may be achieved by making a special construction for the nozzle of the syringe, thus allowing for direct coupling of the tube and the syringe. Alternatively, the tube may be fitted with a female liner or luer lock connector. With this connector, the tube can be fitted to the syringe via the male luer or luer lock connector on the syringe.

In another alternative embodiment, the barrel of the syringe and the sealable tube may be integrated and may be made in one piece.

This apparatus is packaged and sterilized by means of gas or radiation.

In method terms, the invention consists in a method for filling the above apparatus with a medical fluid comprising:
bringing into fluid communication via sterile docking: said syringe with the sealable tube and a container with a medical fluid, said container being fitted with a sealable tube that is closed on one end and that is in communication with the fluid on the other end,
introducing said medical fluid in said syringe,
sealing the tube between said syringe and said container and
cutting said seal.

The above objects, features and advantages of our invention will become more readily apparent from the following description and drawings.

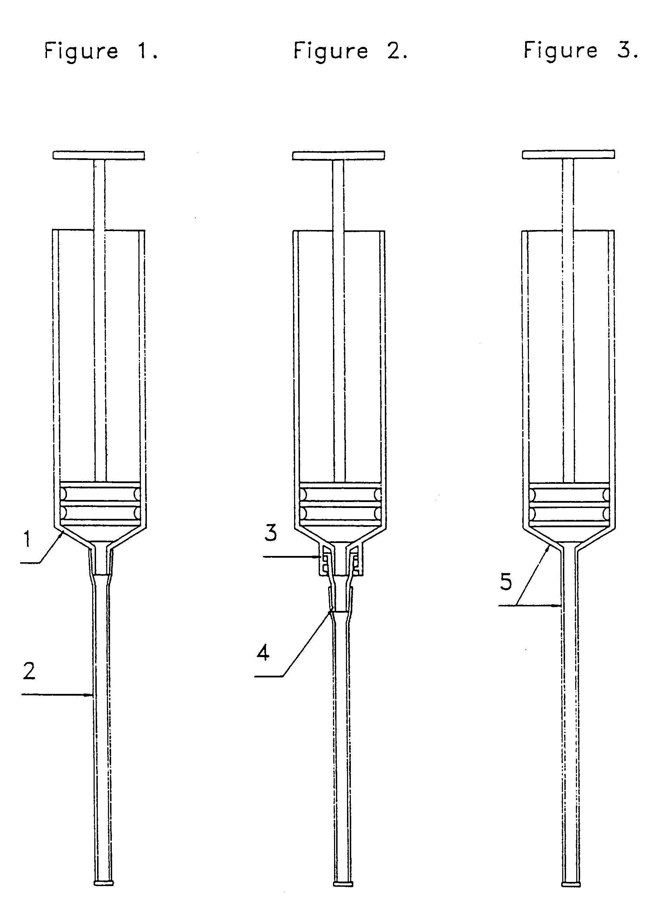

Figure 1 shows a syringe (1) with the closed, sealable tube (2) attached to the syringe via a direct coupling. The tube can e.g. be attached via welding, glueing or clamping. For that purpose, the nozzle of the syringe can be fitted with a hose coupling. The sealable tube may be fitted with a sleeve to prevent disconnection of the tube due to high pressures such as may be generated by some syringe pumps.
Apart from the nozzle, the syringe may be of any known design and/or material and should preferably comply with all the other ISO specifications.
The tube can be made of a flexible, sealable material like e.g. PVC or polyethylene.

Figure 2 shows a syringe according to ISO, where the closed, sealable tube is connected to the male luer lock connector of the syringe (3) via a female liner lock connector (4) on the tube.
This connection may be equipped with a tamper evidence for reasons of proof of e.g. sterility. This tamper evidence may e.g. be a seal, or a piece of shrink-foil around the connectors or a breakable catch.

Figure 3 shows a syringe with an integrated tube (5), where the syringe and the tube are both made in one piece. They may be made from materials like e.g. polyethylene.

In the following figures, several additions to this basic concept are shown.

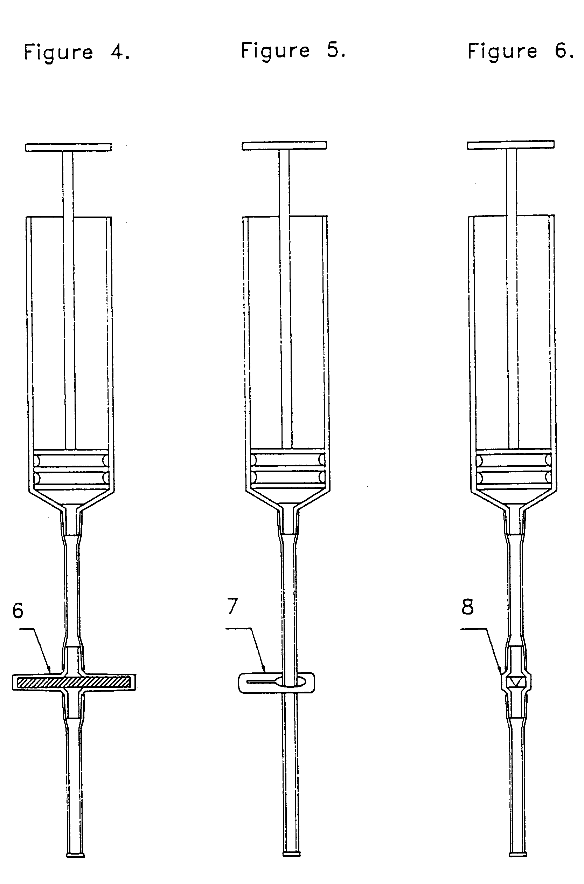

Figure 4 shows a tube fitted with a filter (6) for the removal of micro-organisms or particles from the fluid with which the syringe is filled. Generally, a filter with a pore size of 0.2 »m or larger is chosen for this purpose.

Figure 5 shows a tube fitted with a clamp (7). A clamp may be used to temporarily shut off the flow of fluid through the tube.

Figure 6 shows a tube fitted with a one-way check-back valve (8) allowing only fluid flow in the direction of the syringe. Such a valve will prevent contamination of the fluid in the container with a product that is contained in the syringe. Such a valve might be used if different fluids from more than one container must be sampled in the same syringe to prevent accidental mixing of the solution in the second container that is being sampled.

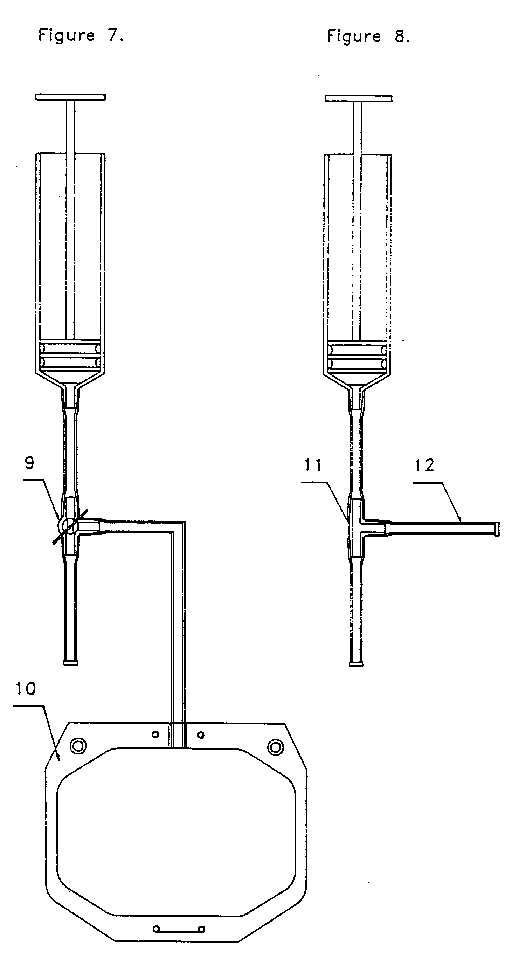

Figure 7 shows a tube fitted with a 3-way valve (9) with an empty container (10). In the tube, a 3-way valve may be fitted. This valve may allow communication between the syringe and either of two pieces of closed sealable tube.
One of these tubes can be used to connect with the solution to be sampled via sterile docking. After sampling, by turning the plug of the valve, open fluid communication is accomplished between the syringe and the other tube. To this tube a component can be coupled with the aid of sterile docking.
For instance, a flexible container (10), as shown in the figure 7, may be connected to this tube. Then, this container can be used as e.g. a waste bag to collect the air that is expelled from the syringe. Use of this 3-way valve in this configuration therefore makes it possible to expel the air from the syringe in a closed system. This may be especially important in the preparation of cytotoxic drugs to prevent aerosols and spillage. It may also be important to keep the container that is being sampled essentially free of air, which may be important if the container that is being sampled contains a blood-product or if the fluid is easily oxidized.
Also, such a bag may serve as a transfer bag or as a mixing bag to collect several portions of fluid which need subsequent mixing.
Howevever, any other component containing a sealable tube can be coupled to this 3-way valve.

Figure 8 shows a tube fitted with a y-piece or t-shaped piece (11) and a second closed sealable tube (12). This tube can be used for the connection of other components with the aid of sterile docking. It may e.g. be connected to a flexible container that can serve as a transfer-bag or as a waste-bag or as a mixing-bag as described above.

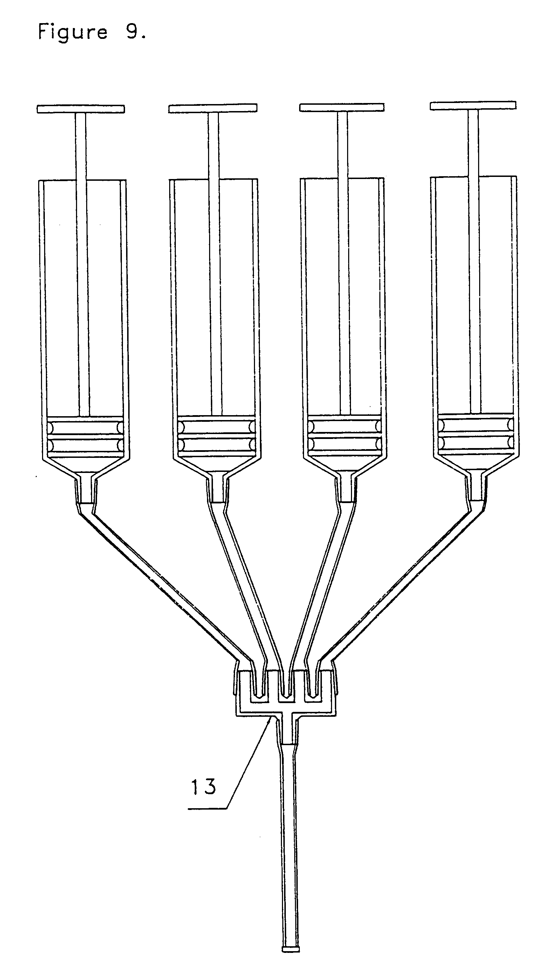

Figure 9 shows a manyfold consisting of 4 apparatus's being in communication with one closed, sealable tube via a more-way connector (13).
One manyfold might e.g. contain 4 or 10 syringes. After sterile docking to a bulk container with a medical solution, these syringes are filled, sealed and stored until use. This method of filling of syringes may be used e.g. in the preparation of parenteral nutrition for neonates in which usually at least one liter of solution must be compounded from which approx. 20 syringes can be filled at the same time. After filling, these syringes can be disconnected via sealing and can be stored until needed.

In another alternative embodiment (not shown), the tube and the syringe may be fitted with some form of identification.
For instance, the syringe and the tube may be fitted with labels or flag-labels or may be printed with a unique code.

The tube may be fitted with a number of identical identification codes and the syringe -if the syringe and the tube can be disconnected- may be fitted with at least one code with which the syringe and the tube can be hatched. Identification codes consisting of numbers are in use on the tubes of standard bloodbags to allow for a positive identification of the bag and samples taken from it. If a part of the tube of the syringe is taken as a sample for testing, positive identification with the syringe from which the sample was taken is possible. Also, after filling of the syringe from the container, a piece of tube with such an identification code can be left on the tube of the container for a positive match of the container and the syringe with the medical fluid. This may especially be important for the administration of blood products which are filled from a container and subsequently tested for compatability with the patient's blood. Use of a positive means of identification may prevent an accidental break in administrative procedures and thereby increase the safety of blood transfusion. Also, for the administration of drug solutions, filled from a bulk-container, such identification codes might improve the safety.

Also, any combination of the above mentioned embodiments forms part of this invention.

These apparatus's are packaged and sterilized and subsequently provided to the user.

A sterile, empty syringe with a sealable tube according to the present invention, is brought in open fluid communication with a medical solution in a container with a sealable tube via sterile docking. This solution may e.g. be a standard IV solution or it may be a diluted solution of a drug or it may be a bloodproduct.
The tube on the syringe and the tube on the container may e.g. be made of a material like PVC or an other polymer that can be sealed.
Both sealable tubes are pint in an apparatus for sterile docking. The tubes are docked and the dock is opened, thereby creating fluid communication between the container with the medical fluid and the syringe. The fluid is drawn into the syringe. Air is expelled from the syringe and the syringe is filled with the exact amount of fluid required.
Now, the tube is sealed. There are several methods for sealing tubes such as heat-sealing or RF-welding. An apparatus for sealing tubes is marketed in the U.S. by the Sebra company. Several patents describe these sealing techniques such as U.S. patent no's 4.013.860 and 4.186.292 and 4.390.832.

Then, the seal is cut producing a syringe with a medical fluid and a closed tube and a container with a closed tube.
The tube on the container can be used again to fill other syringes with the medical solution from the container.
The syringe may then be labelled and transported to the ward as in the usual procedure.
If the fluid in the syringe must be administered to the patient via an administration system or needle with a female liner or luer lock, the luer-type syringe should be used. Before administration, the remaining sealable tube with the female luer connector should be removed, thus exposing the male luer connector on the syringe.
For direct IV push injection into a vein or into a running administration system , the syringe may then be coupled to the female liner connector of an injection needle and the fluid can be injected into the patient.
For slow administration via a syringe pump, the connector of the syringe may be coupled to a female liner lock connector on an administration system or on a side-line which is connected to the administration system of the patient.

If the syringe need not be connected to an administration system of the patient by means of a female luer connector, either the embodiment in which the syringe has a direct connection with the tube or the embodiment in which the syringe and the tube are integrated in one piece, is preferably used. Via sterile docking, a tube can be connected to the tube on the syringe. This tube may contain a break-away connector or a clamp and a connector for a needle. The needle can be pierced through an injection port of the patient's administration system. After placing the syringe in the pump and breaking the break-away connector, the fluid can be administered to the patient. This method of working is especially useful in the treatment of patients with cytotoxic drugs because it prevents the occurrence of aerosols and drips during connection of the syringe to the patient's administration system at the bedside.

Referring to the background of this patent-application, it will be clear that the present invention offers many advantages over the prior art for filling a syringe in an open system.
Use of a closed system prevents contamination of the solution in the syringe and of the solution in the container that is being sampled, and it prevents contamination of the environment with the product that is sampled. Furthermore, the disadvantages of working with needles are prevented.

Prevention of microbiological contamination of the solution in the syringe will increase the safety and may reduce hospital-acquired infection in the patients.
Also, this will increase the expiry period for a number of products, thus making recycling of non-used doses possible.
Prevention of microbiological contamination of the solution in the container that is sampled will increase the expiry period for these products, which is an advantage from an economic point of view. Furthermore, the increased expiry also presents an advantage from a medical point of view as the same solution can be sampled more times for the same patient over a period of days or even weeks. This is especially important for patients that need small quantities of bloodproducts to prevent transfusion-related reactions.

Prevention of contamination of the environment with the product that is sampled increases the safety of working with hazardous substances like cytotoxic drugs by reducing the chances of inadvertent exposure to these drugs due to aerosols, needle drips and the like. Furthermore, after sampling, both the syringe and the container -that would contain a punctured rubber membrane in the prior art- are again hermetically closed, sealed products.

Generally, the replacement of needles by a fluid path consisting of two tubes in fluid communication, offers a number of advantages.
In the first place, needle-sticks are prevented.
In the second place, the container can not be punctured accidentally.
In the third place, use of tubes is far more convenient as needles require considerably more force during injection and withdrawal of fluids.
In the fourth place, as no rubber membrane needs to be punctured, no coring or laceration -resulting in particles in the solution- can take place.

## Claims

1. A sterilized apparatus for medical use consisting of a syringe (1) that is fitted with a sealable tube (2), said sealable tube (2) being closed on one end and in communication with said syringe (1) on the other end.

2. The apparatus as defined in claim 1, wherein said sealable tube (2) is in communication with said syringe (1) on said other end via a direct coupling of the syringe (1) to the sealable tube (2).

3. The apparatus as defined in claim 1, wherein said sealable tube (2) is in communication with said syringe (1) on said other end via a female luer or liner lock connector (4) that is fitted on the male liner or luer lock connector (3) of the syringe (1).

4. The apparatus as defined in claim 1, wherein said sealable tube (2) is in open communication with said syringe (1) via an integrated construction in which the barrel of the syringe (1) and the sealable tube (2) are manufactured in one piece (5).

5. The apparatus as defined in any one of the claims 1 to 4, further comprising a filter (6) in said tube (2) for the removal of particles and/or micro-organisms from the solution,

6. The apparatus as defined in any one of the claims 1 to 4, further comprising a clamp (7) on said tube (2) to shut off flow therethrough.

7. The apparatus as defined in any one of the claims 1 to 4, further comprising a one-way check-back valve (8) in said tube (2) permitting only flow toward said syringe (1).

8. The apparatus as defined in any one of the claims 1 to 4, further comprising a three-way valve (9) in said tube (2) the third leg of said valve (9) being connected to a second closed sealable tube that can be used for sterile docking.

9. The apparatus as defined in claim 8, further comprising a flexible container (10) in open communication with said third leg of said three-way valve (9).

10. The apparatus as defined in any one of the claims 1 to 4, further comprising a Y-piece (11) In said tube (2) the third leg of said Y-piece (11) being in communication with a second closed sealable tube (12).

11. The apparatus as defined In claim 10, further comprising a flexible container in communication with said third leg of said Y-piece (11).

12. A multitude of apparatus's as defined in any one of the claims 1 to 4, connected to one piece of closed sealable tubing via a more-way connector (13) between the tubes connected to the syringes (1) and said tube, creating a manyfold.

13. The apparatus as defined in any one of the claims 1 to 4, wherein said tube (2) is provided with at least two identifying codes.

14. The apparatus defined in claim 3, further comprising a tamper evidence between said male and said female liner or luer lock connector (3, 4).

15. A method for filling the apparatus of claim 1 with a medical fluid comprising:
bringing into fluid communication via sterile docking: said syringe (1) with the sealable tube (2) and a container with a medical fluid, said container being fitted with a sealable tube that is closed on one end and that is in communication with the fluid on the other end,
introducing said medical fluid in said syringe (1),
sealing the tube (2) between said syringe (1) and said container and
cutting said seal.

## Patentansprüche

1. Sterilisierte Vorrichtung für medizinische Zwecke, bestehend aus einer Spritze (1), die mit einem versiegelbaren Schlauch (2) ausgestattet ist, wobei der genannte versiegelbare Schlauch (2) an einem Ende geschlossen ist und am anderen Ende eine Verbindung mit der genannten Spritze (1) hat.

2. Vorrichtung nach Anspruch 1, bei der der genannte versiegelbare Schlauch (2) mit der genannten Spritze (1) an dem genannten anderen Ende über direktes Koppeln dar Spritze (1) mit dem versiegelbaren Schlauch (2) in Verbindung ist.

3. Vorrichtung nach Anspruch 1, bei der der genannte versiegelbare Schlauch (2) mit der genannten Spritze (1) an dem genannten anderen Ende über einen Aufsteck-Lueranschluß oder eine Aufsteck-Luer-Sperre (4) in Verbindung steht, der mit dem Einsteck-Luer oder der Einsteck-Luer-Sperre (3) an der Spritze (1) verbunden ist.

4. Vorrichtung nach Anspruch 1, bei der sich der genannte versiegelbare Schlauch (2) über eine integrierte Konstruktion, in der der Zylinder der Spritze (1) und der versiegelbare Schlauch (2) aus einem Stück (5) gefertigt sind, in einer offenen Verbindung mit der genannten Spritze (1) befindet.

5. Vorrichtung nach einem der Ansprüche 1 bis 4, ferner ein Filter (6) in dem genannten Schlauch (2) enthaltend, zum Abfiltern von Partikeln und/oder Mikroorganismen aus der Lösung.

6. Vorrichtung nach einem der Ansprüche 1 bis 4, ferner eine Klemme (7) an dem genannten Schlauch (2) umfassend, um einen Durchfluß durch diesen abzusperren.

7. Vorrichtung nach einem der Ansprüche 1 bis 4, ferner ein Einweg-Rückschlagventil (8) in dem genannten Schlauch (2) umfassend, das einen Fluß nur in Richtung der genannten Spritze (1) zuläßt.

8. Vorrichtung nach einem der Ansprüche 1 bis 4, ferner ein Drei-Wege-Ventil (9) in dem genannten Schlauch (2) umfassend, wobei der dritte Leitungszweig des genannten Ventils (9) an einen zweiten geschlossenen, versiegelbaren Schlauch angeschlossen ist, der für das sterile Andocken verwendet werden kann.

9. Vorrichtung nach Anspruch 8, ferner einen flexiblen Behälter (10) umfassend, der mit dem genannten dritten Leitungszweig des genannten Drei-Wege-Ventils (9) in offener Verbindung steht.

10. Vorrichtung nach einem der Ansprüche 1 bis 4, ferner ein Y-Stück (11) in dem genannten Schlauch (2) umfassend, wobei der dritte Leitungszweig des genannten Y-Stücks (11) mit einem zweiten geschlossenen, versiegelbaren Schlauch (12) in Verbindung steht.

11. Vorrichtung nach Anspruch 10, ferner einen flexiblen Behälter umfassend, der mit dem genannten dritten Leitungszweig des genannten Y-Stücks (11) in Verbindung steht.

12. Eine Vielzahl von Vorrichtungen nach einem der Ansprüche 1 bis 4, die an einen geschlossenen, versiegelbaren Schlauch angeschlossen sind, über einen Mehrweganschluß (13) zwischen den Schläuchen, die an die genannte Spritze (1) angeschlossen sind, und dem genannten Schlauch, wodurch ein Verteiler gebildet wird.

13. Vorrichtung nach einem der Ansprüche 1 bis 4, bei der der genannte Schlauch (2) mit mindestens zwei kennzeichnenden Codes ausgestattet ist.

14. Vorrichtung nach Anspruch 3, ferner zwischen demgenannten Einsteck- und dem genannten Aufsteck-Luer bzw. der entsprechenden Luer-Sperre (3, 4) eine Sicherheitsvorrichtung umfassend.

15. Methode zum Füllen der Vorrichtung nach Anspruch 1 mit einer medizinischen Flüssigkeit, die folgendes umfaßt:
Über das sterile Andockverfahren werden folgende Teile in eine Flüssigkeitsverbindung gebracht: die genannte Spritze (1) mit dem versiegelbaren Schlauch (2) und einem Behälter mit einer medizinischen Flüssigkeit, wobei der genannte Behälter mit einem versiegelbaren Schlauch ausgestattet ist, der an einem Ende geschlossen ist und am anderen Ende mit der Flüssigkeit in Verbindung steht,
Einbringen der genannten medizinischen Flüssigkeit in die genannte Spritze (1),
Versiegeln des Schlauchs (2) zwischen der genannten Spritze (1) und dem genannten Behälter und
Durchtrennen der genannten Versiegelung.

## Revendications

1. Dispositif stérilisé à usage médical comprenant une seringue (1) qui est pourvue d'un tube (2) apte à être scellé, ledit tube apte à être scellé étant fermé à une extrémité et en communication avec ladite seringue à l'autre extrémité.

2. Dispositif tel que défini dans la revendication 1, dans lequel ledit tube apte à être scellé (2) est en communication avec ladite seringue (1) à ladite autre extrémité par l'intermédiaire d'un couplage direct de la seringue (1) sur le tube apte à être scellé (2).

3. Dispositif tel que défini dans la revendication 1, dans lequel ledit tube apte à être scellé (2) est en communication avec ladite seringue (1) à ladite autre extrémité par l'intermédiaire d'un embout femelle ou d'un connecteur de verrouillage d'embout (4) qui est fixé sur l'embout mâle ou le connecteur de verrouillage d'embout (3) de la seringue (1).

4. Dispositif tel que défini dans la revendication 1, dans lequel ledit tube apte à être scellé (2) est en communication ouverte avec ladite seringue (1) par l'intermédiaire d'une construction intégrée dans laquelle le cylindre de la seringue (1) et le tube apte à être scellé sont fabriqués d'un seul tenant (5).

5. Dispositif tel que défini selon l'une quelconque des revendications 1 à 4, comprenant en outre un filtre (6) dans ledit tube (2) pour l'enlèvement de particules et/ou de micro-organismes de la solution.

6. Dispositif tel que défini selon l'une quelconque des revendications 1 à 4, comprenant en outre une agrafe (7) sur ledit tube (2) pour y bloquer la circulation.

7. Dispositif tel que défini selon l'une quelconque des revendications 1 à 4, comprenant en outre une valve antiretour unidirectionnelle (8) dans ledit tube (2), permettant uniquement un écoulement vers ladite seringue (1).

8. Dispositif tel que défini selon l'une quelconque des revendications 1 à 4, comprenant en outre une valve (9) à trois voies dans ledit tube (2), la troisième branche de cette valve étant connectée à un second tube formé apte à être scellé qui petit être utilisé pour une fixation stérile.

9. Dispositif selon la revendication 8, comprenant en outre un conteneur flexible (10) en communication ouverte avec ladite troisième branche de ladite valve à trois voies (9).

10. Dispositif selon l'une quelconque des revendications 1 à 4, comprenant en outre une pièce en Y (11) dans ledit tube (2), la troisième branche de ladite pièce en Y (11) communiquant avec un second tube fermé apte à être scellé (12).

11. Dispositif selon la revendication 10, comprenant en outre un conteneur flexible communiquant avec ladite troisième brandie de ladite pièce en Y (11).

12. Une pluralité de dispositifs tels que définis dans l'une quelconque des revendications 1 à 4, connectés à une pièce de tubage apte à être scellée fermée par l'intermédiaire d'un connecteur à plus de voies (13) entre les tubes connectés aux tubes (1) et ledit tube créant un collecteur.

13. Dispositif tel que défini dans l'une quelconque des revendications 1 à 4, dans lequel ledit tube (2) est pourvu d'au moins deux codes d'identification.

14. Dispositif défini dans la revendication 3, comprenant en outre une preuve de manipulation entre ledit embout mâle et femelle ou connecteur d'embout de verrouillage (3,4).

15. Procédé pour remplir le dispositif selon la revendication 1 avec un fluide médical, comprenant les étapes consistant à:
amener en communication fluidique par l'intermédiaire d'une fixation stérile: ladite seringue (1) avec le tube apte à être scellé (2) et un conteneur avec un fluide médical, ledit conteneur étant pourvu d'un tube apte à être scellé qui est fermé à une extrémité et qui est en communication avec le fluide à l'autre extrémité,
introduire ledit fluide médical dans ladite seringue (1),
sceller le tube (2) entre ladite seringue (1) et ledit conteneur et
rompre ledit scellé.
